# FASCICULE DE BREVET EUROPEEN

(11) **EP 3 641 589 B1**
(45) Date de publication et mention de la délivrance du brevet: **20.04.2022**
(21) Numéro de dépôt: 18748992.7
(22) Date de dépôt: 22.06.2018
(51) Int. Cl.: A45D 37/00, A45D 40/00, A45D 40/24

(54) **CAPSULE DÉFORMABLE À USAGE UNIQUE**
VERFORMBARE EINWEGKAPSEL
SINGLE-USE DEFORMABLE CAPSULE

(30) Priorité: 23.06.2017 FR 1755751
(43) Date de publication de la demande: 29.04.2020
(73) Titulaire: LABORATOIRES M&L, 04100 Manosque (FR)
(72) Inventeur: DE BRUGIERE, Philippe, 78160 Marly Le Roi (FR)
(74) Mandataire: Derambure Conseil
(86) Numéro de dépôt international: PCT/FR2018/051519
(87) Numéro de publication internationale: WO 2018/234717

(56) Documents cités:
- WO-A1-03/076000
- WO-A1-2014/083279
- WO-A1-2016/102816
- WO-A2-2004/069731
- FR-A- 1 271 583
- FR-A1- 2 894 942

## Description

### DOMAINE DE L'INVENTION

L'invention concerne le domaine des capsules déformables à usage unique.

### CONTEXTE DE L'INVENTION

Selon xyz un premier art antérieur, il est prévu quantité de structures différentes pour une capsule contenant un produit cosmétique prêt à l'emploi, ou bien pour une capsule contenant deux phases séparées l'une de l'autre, mais à mélanger entre elles pour obtenir le produit cosmétique ensuite directement prêt à l'emploi.

Le contexte d'utilisation de ces capsules est assez différent du contexte dans lequel deux capsules contenant chacune une seule phase d'un produit cosmétique, non utilisables seules indépendamment l'une de l'autre, doivent avoir leurs contenus mélangés ensemble et homogénéisés entre eux, avant que ce mélange homogénéisé des deux phases donne le produit cosmétique prêt à l'emploi.

Selon un deuxième art antérieur, par exemple décrit dans la demande de brevet FR 1459620, il est prévu des capsules de forme plutôt ovoïde. Ces capsules contiennent chacune une phase d'un produit cosmétique formé par le mélange homogénéisé de ces deux phases. Le mélange est bien homogénéisé, et le produit cosmétique résultant est prêt à l'emploi immédiatement.

Un troisième art antérieur, décrit dans la demande FR2894942, divulgue un dispositif de conditionnement et de distribution de produits à mélanger.

L'invention propose d'améliorer la disposition des deux fonctions de la machine à mélanger, qui sont d'une part la fonction d'expulsion alternative du contenu de chacune des capsules à destination de l'autre capsule, et vice-versa ensuite, et d'autre part la fonction de chauffage des contenus de ces deux capsules, ce qui améliore la compacité de la machine à mélanger les contenus des deux capsules, tout en utilisant une structure simple de chacune des capsules.

Préférentiellement, la compacité de l'ensemble des deux capsules une fois assemblées l'une à l'autre va être également améliorée, ce qui permettra également, d'améliorer encore la compacité de la chambre de mélange de la machine destinée à mélanger les deux phases respectivement contenues dans les deux capsules, en disposant ces deux capsules en vis-à-vis l'une de l'autre, leurs faces planes étant disposées en regard l'une de l'autre.

### RESUME DE L'INVENTION

Le but de la présente invention est de fournir une capsule palliant au moins partiellement les inconvénients précités.

Plus particulièrement, l'invention vise à fournir une capsule dont la simplicité d'utilisation est améliorée. D'une part, la face plane de la poche permet un chauffage simple et homogène, et même préférentiellement avec un seul élément chauffant de structure simple à épaisseur avantageusement constante, lorsque deux capsules sont disposées en vis-à-vis l'une de l'autre. D'autre part, la face bombée et souple de la poche permet de manière simple une expulsion complète du contenu de la poche hors de cette poche, par simple pression mécanique, et même préférentiellement avec deux éléments mécaniques, par exemple de type pistons, disposés en vis-à-vis l'un de l'autre, lorsque deux capsules sont disposées en vis-à-vis l'une de l'autre, chaque élément mécanique étant chargé d'exercer à tour de rôle une pression sur l'une des capsules de manière à vider son contenu vers l'autre capsule et vice-versa ensuite.

A cette fin, la présente invention propose une capsule selon la revendication 1.

De préférence, la poche comprend deux compartiments séparés par une paroi frangible, ladite phase se trouvant seulement dans le compartiment où ne débouche pas ledit canal, seul ce compartiment comprenant ladite phase étant bombé avant rupture de ladite paroi frangible. Ainsi, cette phase sera bien conservée, par exemple pendant son stockage, car la paroi frangible séparant les deux compartiments de la poche permet un remplissage sous vide et hermétique de la phase dans la poche.

Selon l'invention, il est également prévu un couple de capsules déformables à usage unique, chaque capsule étant selon la revendication 1 .

De préférence, la première capsule et la deuxième capsule sont reliées entre elles par la mise en communication entre le premier canal et le deuxième canal, par leur côté débouchant à l'extérieur de leur capsule, l'intérieur des premier et deuxième canaux forme un espace situé entre les première et deuxième poches et qui, étendu à l'intérieur des première et deuxième poches, est : séparé, par une première paroi frangible, d'un compartiment de la première poche contenant l'une des deux dites phases, séparé, par une deuxième paroi frangible, d'un compartiment de la deuxième poche contenant l'autre des deux dites phases, vide, avant la rupture des première et deuxième parois frangibles.

Suivant des modes de réalisation préférés, l'invention comprend une ou plusieurs des caractéristiques suivantes qui peuvent être utilisées séparément ou en combinaison partielle entre elles ou en combinaison totale entre elles, avec l'un des objets précités de l'invention.

De préférence, la portion mâle ou femelle de connecteur destinée à s'accoupler avec la portion femelle ou mâle de connecteur d'une autre capsule est en polymère injecté, avantageusement en polypropylène ou en polyéthylène.

Ainsi, l'accouplement des capsules entre elles est réalisé sans nécessité une trop grande complexification de la structure des capsules à accoupler entre elles.

De préférence, le film est préférentiellement soudé sur cette coque, avantageusement soudé par chauffage thermique.

Ainsi, la fabrication de la capsule est grandement améliorée, car toutes les formes sont reportées et mises en place sur l'une des faces de la capsule, tandis que l'autre face de la capsule, entièrement plane se contente de recouvrir la première face de la capsule laquelle première face est en relief. De plus, la réalisation de la forme dissymétrique de la poche de chaque capsule est réalisée de manière simple tout en obtenant une poche de capsule qui restera robuste.

Ainsi, la simplicité de fabrication est améliorée, car le film, qui est plan, recouvre simplement une coque, qui est en relief mais qui est préformée. Une seule structure, à savoir une coque recouverte par un film, permet de réaliser simultanément aussi bien la poche de la capsule que la portion de canal de communication partant de cette poche de capsule.

De préférence, la coque thermoformée est multicouche et comprend avantageusement une couche interne en polymère polyoléfine, le film est multicouche et comprend avantageusement une couche interne en polymère polyoléfine.

Le polymère polyoléfine est un matériau qui est particulièrement bien adapté à être soudé sur lui-même.

De préférence, la capsule comprend une zone tampon vide séparée de la poche par une paroi frangible, aucun constituant cosmétique ne se trouvant dans ladite zone tampon, ledit canal débouche d'un côté au niveau de la zone tampon et de l'autre côté à l'extérieur de la capsule.

Ainsi, d'une part une bonne conservation des phases du produit cosmétique est assurée avant leur mélange, car la paroi frangible séparant les deux compartiments de la poche permet un remplissage sous vide et hermétique de la phase dans la poche, et d'autre part l'opération de mélange elle-même est substantiellement simplifiée, car la poche de la capsule, qui ne contenait initialement qu'une seule phase, peut maintenant recevoir l'ensemble du mélange contenant les deux phases à mélanger entre elles pour obtenir le produit cosmétique prêt à l'emploi.

De préférence, ledit canal est de section constante sur la majeure partie de sa longueur, de préférence sur la totalité de sa longueur.

Ainsi, le couplage de l'accélération du mélange lors de la traversée du canal avec la chute de pression qui l'accompagne, suivie d'une décélération du mélange avec la remontée de pression qui l'accompagne, favorise le cisaillement du mélange après cette traversée, et par conséquent améliore l'homogénéisation de ce mélange entre les contenus des poches de deux capsules distinctes.

De préférence, ladite partie de canal à section constante présente une section comprise entre 0.5 et 3 mm², de préférence entre 1 et 2 mm ².

Ainsi, cette faible valeur de section de canal accélère le mélange traversant le canal et fait chuter sa pression, améliorant alors le cisaillement du mélange sortant du canal, cisaillement qui, devenant plus important, améliore l'homogénéisation du mélange entre les contenus des poches de deux capsules distinctes.

De préférence, ledit canal présente une taille ou un diamètre qui vaut au plus 20% de la taille ou du diamètre de la poche, de préférence au plus 15%, encore plus de préférence au plus 12%. De préférence, ledit canal présente une taille ou un diamètre qui vaut au moins 2% de la taille ou du diamètre de la poche, de préférence au moins 4%, encore plus de préférence au moins 8%.

Ces valeurs de ratios entre d'une part la taille du canal et d'autre part la taille de la poche assurent d'une part une valeur maximum préférentielle du seuil relatif de rétrécissement permettant d'obtenir un seuil particulièrement intéressant de cisaillement et donc d'homogénéisation et d'autre part une valeur minimum préférentielle du seuil relatif de rétrécissement permettant de maintenir un temps de passage avantageusement court pour effectuer le transfert du mélange d'une poche d'une capsule à l'autre poche de l'autre capsule.

De préférence, l'épaisseur de la poche est comprise entre 5% et 30% de sa longueur, de préférence entre 10% et 20% de sa longueur. De préférence, l'épaisseur de la poche est comprise entre 10% et 50% de sa largeur, de préférence entre 20% et 40% de sa largeur.

Ces ratios de dimensions facilitent la déformation de la poche de la capsule, sans avoir besoin d'utiliser un matériau mou ou de faible épaisseur. Cette déformation augmente alors temporairement le ratio volume sur surface de la poche, ce qui est utile pour accueillir facilement l'ensemble du mélange traversant le canal et arrivant dans cette poche.

De préférence, ledit rétrécissement en forme de canal est suffisamment étroit et suffisamment long, pour qu'une pression mécanique puisse transférer le contenu d'une desdites poches vers l'autre desdites poches, de manière à réaliser un cisaillement dudit contenu sortant dudit rétrécissement en forme du canal, ledit cisaillement homogénéisant le mélange desdites deux phases, ledit mélange homogénéisé étant un produit cosmétique directement consommable par le consommateur final.

Ainsi est fournie une capsule, à usage unique, de fabrication d'un produit cosmétique, directement consommable par le consommateur final, pour laquelle le mélange obtenu est mieux homogénéisé.

De préférence, le canal, formé par la partie du premier canal et la partie du deuxième canal, à section constante, présente une longueur comprise entre 0.5 et 10 mm, de préférence comprise entre 1 et 5 mm, encore plus de préférence entre 2 et 4 mm.

Ainsi, cet effet combiné d'accélération et de chute de pression, favorisant le cisaillement du mélange sortant du canal et améliorant son homogénéisation, se produit pendant plus longtemps, pendant le temps de traversée du canal.

De préférence, lesdites poches sont chacune suffisamment volumineuse pour contenir tout ledit mélange, de préférence suffisamment volumineux pour contenir une fois et quart tout ledit mélange, encore plus de préférence suffisamment volumineux pour contenir une fois et demie tout ledit mélange. Avantageusement, les poches sont déformables mais pas extensibles.

Ainsi, à chaque transfert du mélange de l'une des poches vers l'autre poche, l'intégralité du mélange traverse le canal, et pas seulement la majorité ou une partie, réduisant alors le nombre de transferts requis pour obtenir un niveau d'homogénéisation donné.

De préférence, ladite première poche à usage unique comprend une quantité prédéterminée d'une phase excipient d'un produit cosmétique, ladite deuxième poche à usage unique comprend une quantité prédéterminée d'une phase contenant des éléments actifs d'un produit cosmétique, ladite deuxième poche étant avantageusement stérilisée. Le mélange juste avant utilisation par le consommateur final d'une phase excipient et d'une phase contenant des éléments actifs et la fabrication d'une monodose à usage instantané évite l'addition de conservateurs, inutiles pour les effets cosmétiques, et habituellement employés en cosmétique conventionnelle pour permettre un stockage du produit cosmétique conventionnel dans le temps.

De préférence, ladite première poche à usage unique comprend une quantité prédéterminée d'une phase grasse d'un produit cosmétique, ladite deuxième poche à usage unique comprend une quantité prédéterminée d'une phase aqueuse d'un produit cosmétique, ladite deuxième poche étant avantageusement stérilisée. Le mélange juste avant utilisation par le consommateur final d'une phase grasse et d'une phase aqueuse et la fabrication d'une monodose à usage instantané évite l'addition de conservateurs, inutiles pour les effets cosmétiques, et habituellement employés en cosmétique conventionnelle pour permettre un stockage du produit cosmétique conventionnel dans le temps.

La phase grasse ou la phase excipient oriente le type de la base soin, tandis que la phase aqueuse ou la phase contenant des éléments actifs constitue majoritairement le complexe d'actifs.

De préférence, ladite phase grasse dudit produit cosmétique peut être de différents types correspondant à différentes galéniques, et ladite phase aqueuse dudit produit cosmétique peut comprendre différents groupes d'éléments actifs correspondant à différents besoins de peau d'un consommateur final. Les différentes galéniques sont par exemple un lait ou une crème. Ainsi, le consommateur final, qui peut tout aussi bien être une consommatrice finale, peut choisir le type de base soin qui lui convient, indépendamment du type de complexe d'actifs qui convient à sa peau.

D'autres caractéristiques et avantages de l'invention apparaîtront à la lecture de la description qui suit d'un mode de réalisation préféré de l'invention, donnée à titre d'exemple et en référence aux dessins annexés.

### BREVE DESCRIPTION DES DESSINS

La figure 1A représente schématiquement une vue en perspective d'un exemple de navette associée à un couple de capsules selon un mode de réalisation de l'invention.
La figure 1B représente schématiquement une vue de côté d'un exemple de navette associée à un couple de capsules selon un mode de réalisation de l'invention.
La figure 2A représente schématiquement une première étape d'association d'un exemple de navette à un couple de capsules selon un mode de réalisation de l'invention, qui est une étape d'insertion du couple de capsules dans la navette.
La figure 2B représente schématiquement une deuxième étape d'association d'un exemple de navette à un couple de capsules selon un mode de réalisation de l'invention, qui est une étape de mise en place du couple de capsules dans la navette.
La figure 2C représente schématiquement une troisième étape d'association d'un exemple de navette à un couple de capsules selon un mode de réalisation de l'invention, qui est une étape de fermeture de la navette contenant le couple de capsules.
La figure 2D représente schématiquement une quatrième étape d'association d'un exemple de navette à un couple de capsules selon un mode de réalisation de l'invention, qui est une étape d'insertion de la navette contenant le couple de capsules dans le mélangeur.
La figure 3A représente schématiquement une vue intérieure pardessus d'une première phase alternative de fonctionnement des organes de pression appuyant alternativement sur l'une ou l'autre des capsules contenues dans la navette disposée dans le mélangeur, selon un mode de réalisation de l'invention.
La figure 3B représente schématiquement une vue intérieure pardessus d'une deuxième phase alternative de fonctionnement des organes de pression appuyant alternativement sur l'une ou l'autre des capsules contenues dans la navette disposée dans le mélangeur, selon un mode de réalisation de l'invention.
La figure 4A représente schématiquement une première étape de disposition du mélange des phases respectivement contenues à l'origine dans les deux capsules du couple de capsules selon un mode de réalisation de l'invention, qui est une étape d'arrêt du mélange auparavant effectué dans le mélangeur.
La figure 4B représente schématiquement une deuxième étape de disposition du mélange des phases respectivement contenues à l'origine dans les deux capsules du couple de capsules selon un mode de réalisation de l'invention, qui est une étape d'extraction, hors du mélangeur, de la navette contenant le mélange.
La figure 4C représente schématiquement une troisième étape de disposition du mélange des phases respectivement contenues à l'origine dans les deux capsules du couple de capsules selon un mode de réalisation de l'invention, qui est une étape d'éloignement de la navette contenant le mélange par rapport au mélangeur.
La figure 5 représente schématiquement une étape d'utilisation du mélange des phases respectivement contenues à l'origine dans les deux capsules du couple de capsules selon un mode de réalisation de l'invention.
La figure 6 représente schématiquement une étape d'évacuation du couple de capsules, selon un mode de réalisation de l'invention, hors de la navette.
La figure 7A représente une vue de dessus d'un exemple de couple de capsules, selon un mode de réalisation de l'invention, assemblées entre elles lors de leur mise en place dans la navette.
La figure 7B représente une vue en perspective d'un exemple de couple de capsules, selon un mode de réalisation de l'invention, assemblées entre elles lors de leur mise en place dans la navette.
La figure 7C représente une vue de dessous d'un exemple de couple de capsules, selon un mode de réalisation de l'invention, assemblées entre elles lors de leur mise en place dans la navette.
La figure 8A représente une vue de côté d'un exemple de couple de capsules, selon un mode de réalisation de l'invention, assemblées entre elles lors de leur mise en place dans la navette.
La figure 8B représente un agrandissement d'une vue de coupe partielle selon l'axe AA d'un exemple de couple de capsules de la figure 7A.
La figure 8C représente un agrandissement d'une vue de coupe partielle selon l'axe BB d'un exemple de couple de capsules de la figure 8A.

### DESCRIPTION DETAILLEE DE L'INVENTION

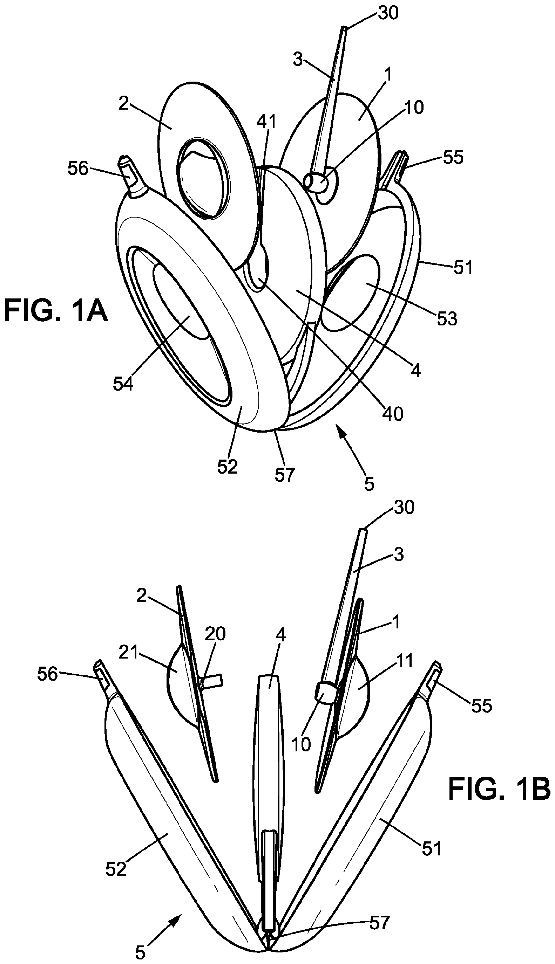

La figure 1A représente schématiquement une vue en perspective d'un exemple de navette associée à un couple de capsules selon un mode de réalisation de l'invention.

La figure 1B représente schématiquement une vue de côté d'un exemple de navette associée à un couple de capsules selon un mode de réalisation de l'invention.

Une première capsule 1 comprend une première poche 11 remplie d'une première phase d'un produit cosmétique, un connecteur mâle 10, un passage de sortie 3 terminé par un orifice de sortie 30.

Une deuxième capsule 2 comprend une deuxième poche 21 remplie d'une deuxième phase d'un produit cosmétique, un connecteur femelle 20.

Le mélange homogénéisé de la première phase et de la deuxième phase donne le produit cosmétique prêt à l'emploi par l'utilisateur. Ce produit cosmétique est par exemple une crème pour le visage entièrement dénuée de conservateurs. La première phase est par exemple une phase aqueuse de type complexe d'actifs, tandis que la deuxième phase est par exemple une phase grasse de type excipient.

Une navette 5 comprend deux coques 51 et 52 articulées en rotation autour d'une charnière 57. La première coque 51 comprend un premier passage 53 en son centre et un premier guide de sortie 55 situé à l'opposé de la charnière 57. La deuxième coque 52 comprend un deuxième passage 54 en son centre et un deuxième guide de sortie 56 situé à l'opposé de la charnière 57. La navette 5 comprend aussi une plaque chauffante 4 qui est rattachée au niveau de la charnière 57, qui est percée en son centre d'un trou 40 destiné à être traversé par le connecteur mâle 10 emboîté dans le connecteur femelle 20, et qui comprend une rainure 41 destinée à recevoir le passage de sortie 3. A la place des passages 53 et 54, les coques 51 et 52 pourraient comprendre simplement des parties souples centrales permettant à des pressions extérieures à la navette 5 de s'exercer sur les poches 11 et 21 des capsules 1 et 2.

Lorsque la navette 5 s'ouvre, les deux coques 51 et 52 s'écartent d'une part l'une de l'autre et d'autre part de la plaque chauffante 4, les deux capsules 1 et 2 peuvent être introduites de part et d'autre de la plaque chauffante 4.

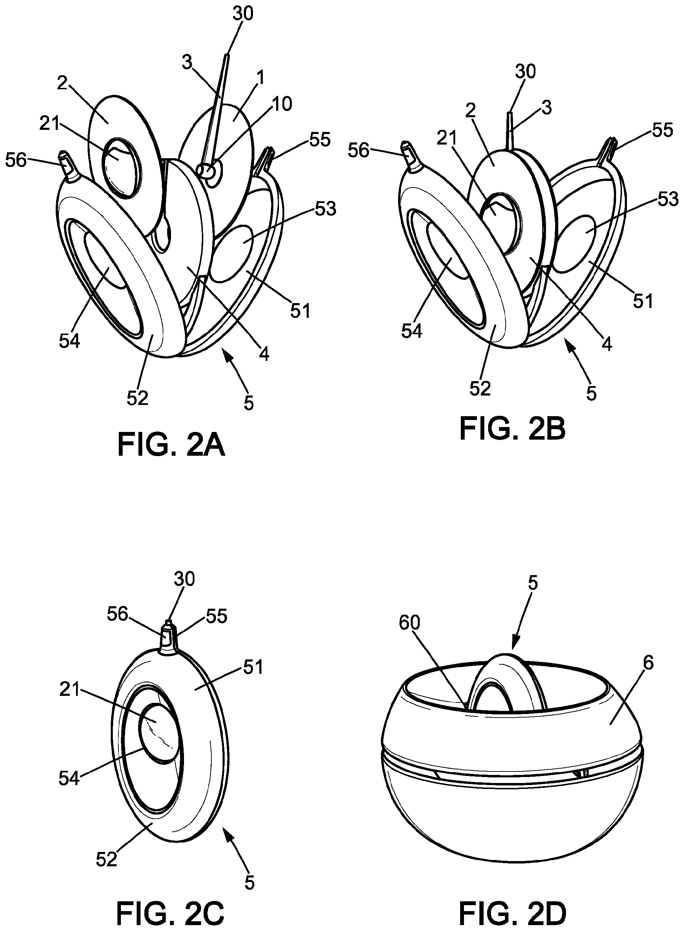

La figure 2A représente schématiquement une première étape d'association d'un exemple de navette à un couple de capsules selon un mode de réalisation de l'invention, qui est une étape d'insertion du couple de capsules dans la navette.

Lorsque la navette 5 s'ouvre, les deux coques 51 et 52 s'écartent d'une part l'une de l'autre et d'autre part de la plaque chauffante 4, les deux capsules 1 et 2 peuvent être introduites de part et d'autre de la plaque chauffante 4, c'est-à-dire respectivement d'une part entre la première coque 51 et la plaque chauffante 4 et d'autre part entre la deuxième coque 52 et la plaque chauffante 4. Cette étape d'insertion du couple de capsules 1 et 2 dans la navette 5 est réalisée manuellement par l'utilisateur.

La figure 2B représente schématiquement une deuxième étape d'association d'un exemple de navette à un couple de capsules selon un mode de réalisation de l'invention, qui est une étape de mise en place du couple de capsules dans la navette.

Les capsules 1 et 2 sont plaquées contre la plaque chauffante 4, de part et d'autre de la plaque chauffante 4. Le connecteur mâle 10 s'emboîte dans le connecteur femelle 20, l'ensemble constitué par les connecteurs mâle 10 et femelle 20 traversant le trou 40, le passage de sortie 3 étant logé dans la rainure 41, l'orifice de sortie 30 débouchant hors de la rainure 41. Cette étape de mise en place du couple de capsules 1 et 2 dans la navette 5 est réalisée manuellement par l'utilisateur.

La figure 2C représente schématiquement une troisième étape d'association d'un exemple de navette à un couple de capsules selon un mode de réalisation de l'invention, qui est une étape de fermeture de la navette contenant le couple de capsules.

Les coques 51 et 52 sont rapprochées l'une de l'autre jusqu'à se toucher et se refermer l'une sur l'autre, par exemple par clipsage l'une dans l'autre. Lorsque la navette 5 contenant le couple de capsules 1 et 2 est fermée, d'une part la première poche 11 traverse le premier passage 53 pour ressortir de la navette 5, d'autre part la deuxième poche 21 traverse le deuxième passage 54 pour ressortir de la navette 5, et par ailleurs le passage de sortie 3 passe entre les guides 55 et 56 pour déboucher par son orifice de sortie 30. Cette étape de fermeture de la navette 5 contenant le couple de capsules 1 et 2 est réalisée manuellement par l'utilisateur.

La figure 2D représente schématiquement une quatrième étape d'association d'un exemple de navette à un couple de capsules selon un mode de réalisation de l'invention, qui est une étape d'insertion de la navette contenant le couple de capsules dans le mélangeur.

La navette 5 est introduite dans un mélangeur 6 dont la fonction va être de mélanger entre elles les première et deuxième phases respectivement contenues dans les capsules 1 et 2, en homogénéisant le mélange, de manière à former par exemple une émulsion qui sera le produit cosmétique final à consommer directement et immédiatement ensuite par l'utilisateur. La navette 5 est introduite dans le mélangeur 6, plus précisément dans une ouverture 60 du mélangeur 6, les guides 55 et 56 ainsi que l'orifice de sortie 30 pénétrant en premier dans l'ouverture 60 du mélangeur 6, de manière à venir se loger et même s'emboîter dans le fond de l'ouverture 60. La navette 5 est poussée au fond de l'ouverture 60 de manière à s'emboîter dans l'ouverture 60 et de manière à être bien maintenue en place dans l'ouverture 60 pendant toute l'opération de mélange qui va se dérouler ensuite. Les guides 55 et 56 refermés l'un sur l'autre et pressés l'un contre l'autre lorsqu'ils sont dans le fond de l'ouverture 60 du mélangeur 6, ont pour effet de pincer le passage de sortie 3 en amont de l'orifice de sortie 30 de manière à ce qu'aucune phase ou aucun produit cosmétique ne puisse sortir de l'espace constitué par le couple de capsules 1 et 2 et par le canal de communication disposé entre ces capsules 1 et 2, tant que la navette 5 est fermée et disposée contre le fond de l'ouverture 60 du mélangeur 6. C'est la fermeture et la pression des guides 55 et 56 l'un contre l'autre, lorsque la navette 5 est fermée et disposée contre le fond de l'ouverture 60 du mélangeur 6, qui entraîne mécaniquement le pincement du passage de sortie 3 en amont de l'orifice de sortie 30. Cette étape d'insertion de la navette 5 contenant le couple de capsules 1 et 2 dans le mélangeur 6 est réalisée manuellement par l'utilisateur.

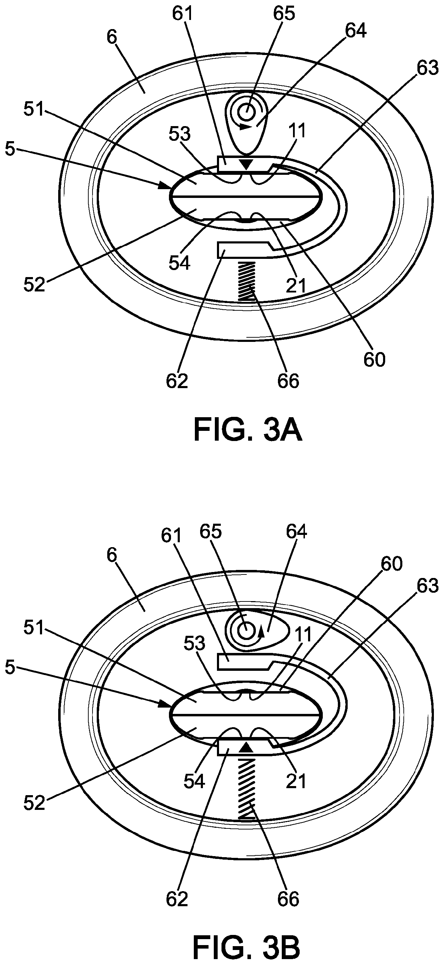

La figure 3A représente schématiquement une vue intérieure pardessus d'une première phase alternative de fonctionnement des organes de pression appuyant alternativement sur l'une ou l'autre des capsules contenues dans la navette disposée dans le mélangeur, selon un mode de réalisation de l'invention.

La figure 3B représente schématiquement une vue intérieure pardessus d'une deuxième phase alternative de fonctionnement des organes de pression appuyant alternativement sur l'une ou l'autre des capsules contenues dans la navette disposée dans le mélangeur, selon un mode de réalisation de l'invention.

Le mélangeur 6 reçoit dans son ouverture 60 la navette 5. En vis-à-vis de la première coque 51, et même plus précisément en regard de la première poche 11 débouchant du premier passage 53, se trouve un premier organe de pression 61, par exemple de type piston ou de type doigt de pression, dont le mouvement latéral (vertical vers le bas sur la figure 3A) indiqué par une flèche triangulaire sur la figure 3A vient presser la première poche 11 de la première capsule 1 pour la vider de son contenu vers la deuxième poche 21 de la deuxième capsule 2 au travers d'un canal de communication inter-capsules non visible sur la figure 3A.

En vis-à-vis de la deuxième coque 52, et même plus précisément en regard de la deuxième poche 21 débouchant du deuxième passage 54, se trouve un deuxième organe de pression 62, par exemple de type piston ou de type doigt de pression, dont le mouvement latéral (vertical vers le haut sur la figure 3B) indiqué par une flèche triangulaire sur la figure 3B vient presser la deuxième poche 21 de la deuxième capsule 2 pour la vider de son contenu vers la première poche 11 de la première capsule 1 au travers d'un canal de communication inter-capsules non visible sur la figure 3B.

Le premier organe de pression 61 et le deuxième organe de pression 62 sont solidaires d'un bras 63. D'un côté de la navette 5, une came oblongue 64 est en appui contre le premier organe de pression 61 pour pouvoir le pousser vers la première coque 51 et vers la première poche 11. De l'autre côté de la navette 5, un ressort 66 en compression est en appui contre le deuxième organe de pression 62 pour pouvoir le pousser vers la deuxième coque 52 et vers la deuxième poche 21. Les mouvements latéraux alternatifs de pression des organes de pression 61 et 62 sur leurs capsules 1 et 2 respectives sont provoqués par la rotation autour d'un axe de rotation 65, dans le sens de la flèche circulaire, de la came oblongue 64.

Lorsque la came oblongue 64 présente sa petite dimension parallèlement au plan moyen de la navette 5, la came oblongue 64 appuie sur le premier organe de pression 61 qui presse la première poche 11 pour la vider de son contenu dans la deuxième poche 21 au travers d'un canal de communication inter-capsules non visible sur les figures 3A et 3B. Le bras 63 se décale d'un côté de la navette 5, écartant de la navette 5 le deuxième organe de pression 62 lequel comprime le ressort 66.

Lorsque la came oblongue 64 présente sa grande dimension parallèlement au plan moyen de la navette 5, le ressort 66 se détend et repousse le deuxième organe de pression 62 qui presse la deuxième poche 21 pour la vider de son contenu dans la première poche 11 au travers d'un canal de communication inter-capsules non visible sur les figures 3A et 3B. Le bras 63 se décale de l'autre côté de la navette 5, écartant de la navette 5 le deuxième organe de pression 62 lequel vient buter contre la came oblongue 64.

Au cours d'un tour de rotation complet de la came oblongue 64, le bras 63 a fait un mouvement de va et vient en translation, le premier organe de pression 61 a d'abord pressé une fois la première poche 11 de la première capsule 1 pour la vider de son contenu vers la deuxième poche 21 de la deuxième capsule 2, le deuxième organe de pression 62 a ensuite pressé une fois la deuxième poche 21 de la deuxième capsule 2 pour la vider de son contenu vers la première poche 11 de la première capsule 1, et le ressort 66 s'est comprimé une fois d'abord puis s'est détendu une fois ensuite. Ce cycle est répété entre 2 et 15 fois, de préférence entre 5 et 10 fois, jusqu'à ce que le mélange entre les deux phases soit bien homogénéisé, par exemple bien émulsionné, de manière à former un produit cosmétique homogène, par exemple une émulsion, prêt à l'emploi direct et immédiat par l'utilisateur, l'utilisateur appliquant par exemple sur la peau de son visage la crème de visage ainsi obtenue, naturelle et fraîche, sans conservateur, puisque le mélange entre phases a été réalisé juste avant utilisation.

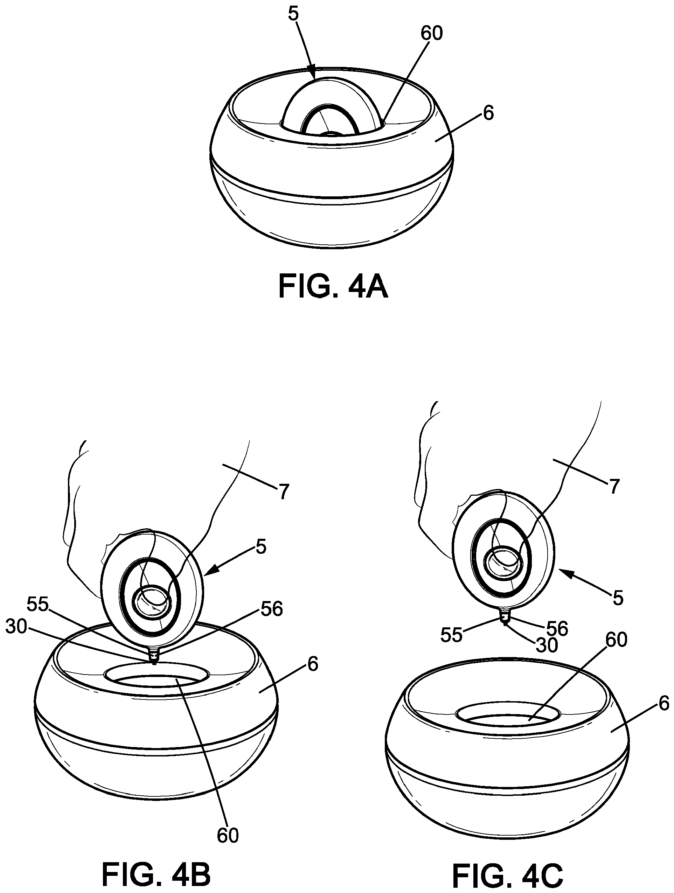

La figure 4A représente schématiquement une première étape de disposition du mélange des phases respectivement contenues à l'origine dans les deux capsules du couple de capsules selon un mode de réalisation de l'invention, qui est une étape d'arrêt du mélange auparavant effectué dans le mélangeur. Une fois bien homogénéisé le mélange entre les deux phases respectivement issues des deux capsules 1 et 2, les organes de pression 61 et 62 arrêtent leur déplacement relativement à la navette 5.

La figure 4B représente schématiquement une deuxième étape de disposition du mélange des phases respectivement contenues à l'origine dans les deux capsules du couple de capsules selon un mode de réalisation de l'invention, qui est une étape d'extraction, hors du mélangeur, de la navette contenant le mélange. La main 7 d'un utilisateur retire la navette 5 de l'ouverture 60 du mélangeur 6. Le retrait de la navette 5 hors de l'ouverture 60 du mélangeur 6 entraîne mécaniquement la libération du pincement de l'orifice de sortie 30 par les guides 55 et 56 des coques 51 et 52.

La figure 4C représente schématiquement une troisième étape de disposition du mélange des phases respectivement contenues à l'origine dans les deux capsules du couple de capsules selon un mode de réalisation de l'invention, qui est une étape d'éloignement de la navette contenant le mélange par rapport au mélangeur. La main 7 d'un utilisateur éloigne la navette 5 du mélangeur 6, pour pouvoir ensuite utiliser le produit cosmétique contenu dans la navette 5, ce produit cosmétique étant le mélange homogénéisé des deux phases respectivement contenues à l'origine dans les deux capsules 1 et 2.

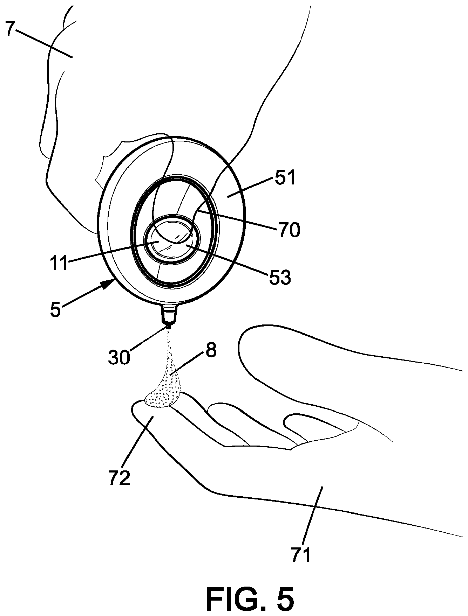

La figure 5 représente schématiquement une étape d'utilisation du mélange des phases respectivement contenues à l'origine dans les deux capsules du couple de capsules selon un mode de réalisation de l'invention.

La main 7 de l'utilisateur, plus précisément un ou plusieurs de ses doigts 70, par exemple son pouce, presse de chaque côté de la navette 5, simultanément au niveau des passages 53 et 54 des coques 51 et 52, sur les poches 11 et 12 des capsules 1 et 2, pour faire sortir le mélange homogénéisé constituant le produit cosmétique 8 prêt à l'emploi par l'orifice de sortie 30 lequel n'est plus pincé. Ce produit cosmétique 8 prêt à l'emploi sort par l'orifice de sortie 30 pour tomber par gravité sur les doigts 72 de l'autre main 71 de l'utilisateur prête à recevoir ce produit cosmétique 8. L'utilisateur disposant de ce produit cosmétique 8 dans sa main 71 peut l'utiliser directement et immédiatement, par exemple étaler la crème de soin 8 sur son visage à l'aide de sa main 71.

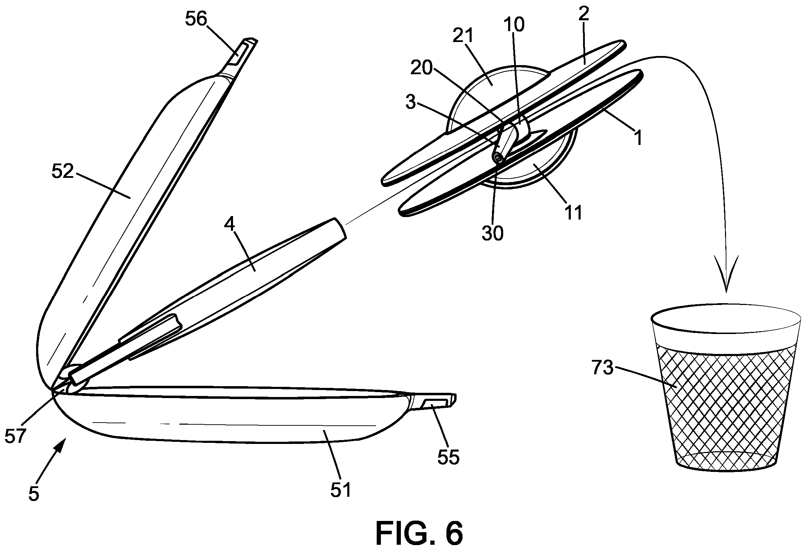

La figure 6 représente schématiquement une étape d'évacuation du couple de capsules, selon un mode de réalisation de l'invention, hors de la navette.

Les coques 51 et 52 ont été écartées l'une de l'autre. L'utilisateur a retiré le couple de capsules 1 et 2 hors de la navette 5, après avoir désolidarisé de la plaque chauffante 4 les capsules 1 et 2. L'utilisateur peut ensuite jeter ce couple de capsules 1 et 2 dans un réceptacle à déchets 73, par exemple dans une poubelle 73.

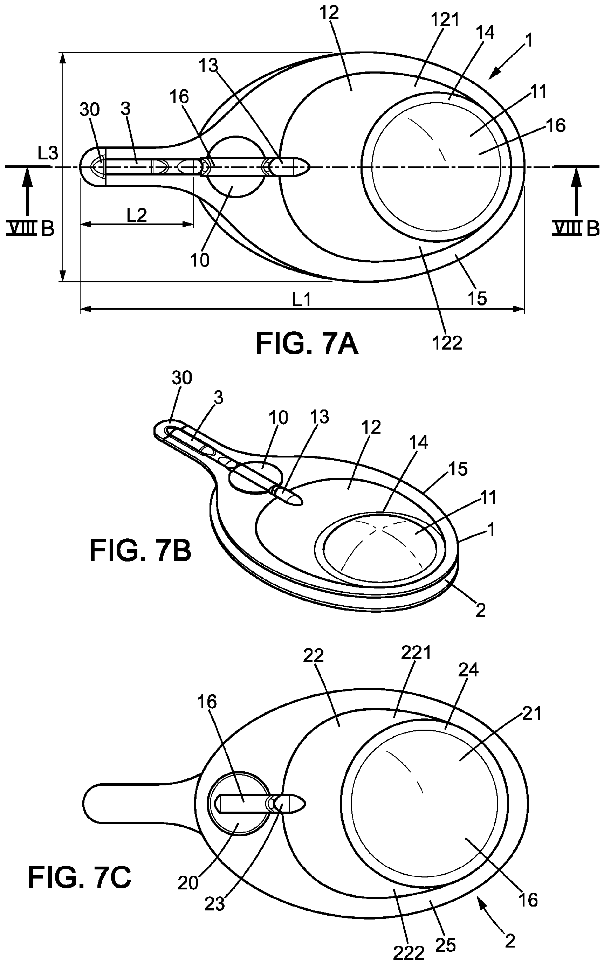

La figure 7A représente une vue de dessus d'un exemple de couple de capsules, selon un mode de réalisation de l'invention, assemblées entre elles lors de leur mise en place dans la navette.

La figure 7B représente une vue en perspective d'un exemple de couple de capsules, selon un mode de réalisation de l'invention, assemblées entre elles lors de leur mise en place dans la navette.

Une première capsule 1 comprend une première poche 11 contenant une première phase destinée à être mélangée à une deuxième phase d'une deuxième poche 21 d'une deuxième capsule 2 pour donner un mélange homogénéisé, par exemple une émulsion, formant le produit cosmétique prêt à l'emploi, directement et immédiatement utilisable par l'utilisateur.

La première capsule comprend aussi une première zone tampon 12. La première poche 11 est séparée de la première zone tampon 12 par une première soudure frangible faible 14. L'ensemble constitué par la première poche 11 et par la première zone tampon 12 est entourée par une première soudure forte 15 nettement plus résistante que la première soudure frangible faible 14.

Lorsque la première soudure faible 14 va être rompue, par une pression exercée sur la première poche 11, la première phase contenue dans la première poche 11 va se déverser d'abord dans la première zone tampon 12 puis dans le tronçon 13 de canal de communication inter-capsules lequel se continue d'une part par un connecteur mâle 10 en direction de la deuxième capsule 2, et d'autre part par un passage de sortie 3 en direction d'un orifice de sortie 30.

L'ensemble des reliefs sur la première capsule 1 sont formés par une coque thermoformée 16. La longueur L1 totale de la première capsule 1 vaut par exemple 70.8mm. La longueur L2 du passage de sortie 3 vaut par exemple 18.15mm. La largeur totale L3 de la première capsule 1 vaut par exemple 36.5mm.

La première capsule 1 déformable à usage unique comprend une quantité prédéterminée d'une première phase d'un produit cosmétique, et comprend également une première poche 11 contenant cette première phase de produit cosmétique, un orifice de sortie 30 débouchant à l'extérieur de la première capsule 1, un canal libre incluant la première zone tampon 12, le tronçon 13 et le passage de sortie 3, et reliant la première poche 11 à l'orifice de sortie 30 en formant un rétrécissement sur une partie de sa longueur, une première paroi frangible 14 séparant la première poche 11 de ce canal libre, toute la première phase de produit cosmétique remplissant toute la première poche 11, la rupture de la première paroi frangible 14 amenant au moins une partie de la première phase de produit cosmétique dans ce canal libre, aucune autre paroi frangible n'étant située dans ce canal libre entre la première poche 11 et l'orifice de sortie 30.

La première capsule 1 déformable à usage unique comprend une première poche 11 contenant une quantité prédéterminée d'une seule des deux phases dont le mélange forme un produit cosmétique prêt à l'emploi, une première zone tampon 12 vide séparée de la première poche 11 par une première paroi frangible 14, aucun constituant cosmétique ne se trouvant dans la première zone tampon 12, une portion de canal incluant d'une part le tronçon 13 et d'autre part le passage de sortie 3 débouchant d'un côté au niveau de la première zone tampon 12 et de l'autre côté à l'extérieur de la première capsule 1 au niveau de l'orifice de sortie 30.

Avant la rupture de la première paroi frangible 14, la première zone tampon vide 12 se trouve dans une position complètement aplatie dans laquelle les parois de la première zone tampon 12 sont disposées l'une contre l'autre.

Après la rupture de la première paroi frangible 14, la première poche 11 s'étend dans la première zone tampon 12 et l'englobe, de manière à augmenter le volume de la première poche 11 alors étendue.

Avantageusement, la première poche 11 présente, dans le plan de l'aplatissement de la première poche 11, lequel est aussi le plan de la figure 7A, une forme générale convexe, et la première zone tampon 12 présente, dans le plan de l'aplatissement de la première poche 11, une forme générale concave complémentaire de la forme générale convexe de la première poche 11. L'ensemble constitué par la première poche 11 et par la première zone tampon 12 présente, dans le plan de l'aplatissement de la première poche 11, une forme générale convexe. La première poche 11 présente, dans le plan de l'aplatissement de la première poche 11, une forme générale circulaire, et la première zone tampon 12 présente, dans le plan de l'aplatissement de la première poche 11, une forme générale en U aux branches 121 et 122 qui s'affinent vers leurs extrémités.

La figure 7C représente une vue de dessous d'un exemple de couple de capsules, selon un mode de réalisation de l'invention, assemblées entre elles lors de leur mise en place dans la navette.

Une deuxième capsule 2 est similaire à la première capsule 1. La deuxième capsule 2 comprend une deuxième poche 21 contenant une deuxième phase destinée à être mélangée à la première phase de la première poche 11 de la première capsule 1 pour donner le mélange homogénéisé, par exemple une émulsion, formant le produit cosmétique prêt à l'emploi, directement et immédiatement utilisable par l'utilisateur.

La deuxième capsule comprend aussi une deuxième zone tampon 22. La deuxième poche 21 est séparée de la deuxième zone tampon 22 par une deuxième soudure frangible faible 24. L'ensemble constitué par la deuxième poche 21 et par la deuxième zone tampon 22 est entourée par une deuxième soudure forte 25 nettement plus résistante que la deuxième soudure frangible faible 24.

Lorsque la deuxième soudure faible 24 va être rompue, par une pression exercée sur la deuxième poche 21, la deuxième phase contenue dans la deuxième poche 21 va se déverser d'abord dans la deuxième zone tampon 22 puis dans le tronçon 23 de canal de communication inter-capsules lequel se continue par un connecteur femelle 20 en direction de la première capsule 1.

L'ensemble des reliefs sur la deuxième capsule 2 sont formés par une coque thermoformée 16. La deuxième zone tampon 22 présente, dans le plan de l'aplatissement de la deuxième poche 21, une forme générale en U aux branches 221 et 222 qui s'affinent vers leurs extrémités.

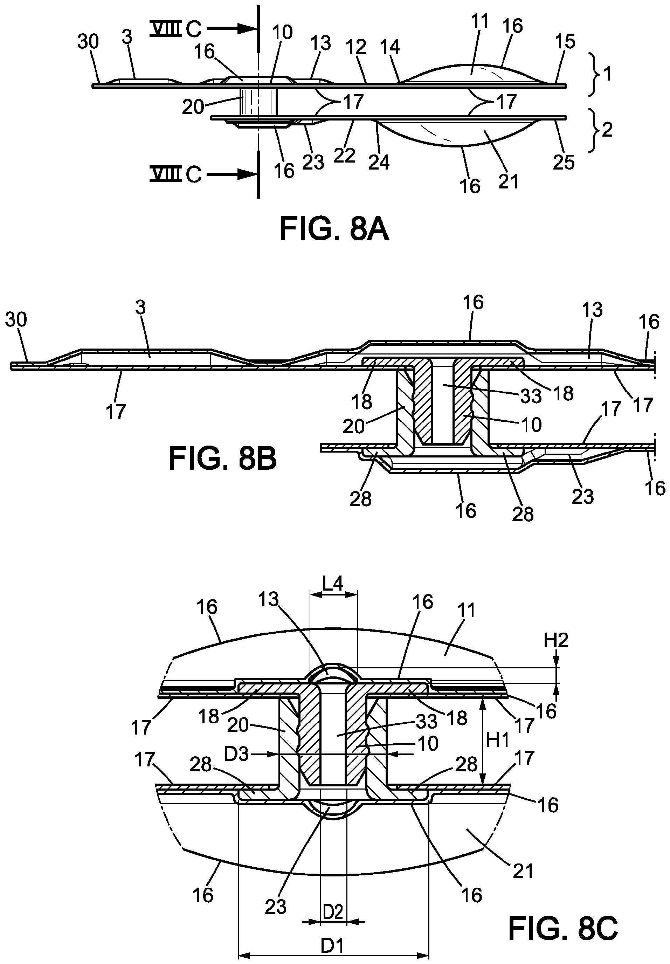

La figure 8A représente une vue de côté d'un exemple de couple de capsules, selon un mode de réalisation de l'invention, assemblées entre elles lors de leur mise en place dans la navette.

La coque thermoformée 16 forme les parties en relief des capsules 1 et 2. Un film plat 17 recouvre la coque thermoformée 16, aussi bien au niveau de la première capsule 1 que de la deuxième capsule 2. Le canal de communication inter-capsules comprend, un premier coude à angle droit formé par le tronçon 13 et le connecteur mâle 10 emboîté dans le connecteur femelle 20, ainsi qu'un deuxième coude à angle droit formé par le connecteur mâle 10 emboîté dans le connecteur femelle 20 et le tronçon 23. La circulation du mélange entre les poches 11 et 21 suit un parcours formant les trois côtés d'un rectangle, le premier grand côté comprenant la première poche 11, la première zone tampon 12, le tronçon 13, le deuxième grand côté comprenant la deuxième poche 21, la deuxième zone tampon 22, le tronçon 23, le petit côté joignant les deux grands côtés précités et comprenant le connecteur mâle 10 emboîté dans le connecteur femelle 20.

Le couple de capsules 1 et 2 assemblées ensemble comprend respectivement deux phases différentes à mélanger pour obtenir un produit cosmétique personnalisé, et comprend deux poches 11 et 21 contenant respectivement les deux phases différentes et étant respectivement incluses dans les deux capsules 1 et 2 et étant reliées entre elles par un canal de communication comprenant au moins une partie de section rétrécie par rapport aux deux poches 11 et 21, cette partie de section rétrécie du canal de communication comprenant deux coudes à angle droit, le premier coude comprenant les parties de canal 13 et 10 à angle droit l'une de l'autre, le deuxième coude comprenant les parties de canal 23 et 10 à angle droit l'une de l'autre. Ces deux coudes à angle droit sont disposés de manière à ce qu'au moins une portion de la partie de section rétrécie du canal de communication comprenant les deux coudes à angle droit forme les trois côtés d'un rectangle.

Chaque capsule 1 ou 2 déformable à usage unique comprend une poche étendue 11 et 12 (ou 21 et 22) contenant une quantité prédéterminée d'une seule des deux phases dont le mélange forme un produit cosmétique prêt à l'emploi, un rétrécissement en forme de canal débouchant d'un côté dans la poche étendue 11 et 12 (ou 21 et 22) et de l'autre côté à l'extérieur de la capsule 1 ou 2. Chaque poche étendue 11 et 12 (ou 21 et 22) est aplatie, l'une de ses deux faces étant plane, l'autre de ses deux faces étant bombée au moins en partie et souple de manière à permettre l'expulsion du contenu de la poche étendue 11 et 12 (ou 21 et 22) par pression mécanique sur elle.

Chaque poche étendue 11 et 12 (ou 21 et 22) comprend deux compartiments 11 et 12 (ou 21 et 22), séparés par une paroi frangible 14 ou 24, la poche proprement dite 11 ou 21 et la zone tampon attenante 12 ou 22 dans laquelle la poche proprement dite 11 ou 21 va pouvoir s'étendre après rupture de la paroi frangible 14 ou 24. La phase se trouvant seulement dans le compartiment 11 ou 21 où ne débouche pas ce canal 13 ou 23, seul ce compartiment 11 ou 21 comprenant la phase est bombé avant rupture de la paroi frangible 14 ou 24. Ainsi, cette phase sera bien conservée, par exemple pendant son stockage, car la paroi frangible 14 ou 24 séparant les deux compartiments 11 et 12 (ou 21 et 22) de la poche étendue 11 et 12 (ou 21 et 22) permet un remplissage sous vide et hermétique de la phase dans la poche 11 et 12 (ou 21 et 22) correspondante.

La figure 8B représente un agrandissement d'une vue de coupe partielle selon l'axe AA d'un exemple de couple de capsules de la figure 7A.

Le connecteur mâle 10 s'emboîte dans le connecteur femelle 20. Le connecteur mâle 10 et le connecteur femelle 20 sont en plastique rigide.

Du côté de la première capsule 1, le connecteur mâle 10 présente une collerette 18 située à sa base dans un plan perpendiculaire à l'axe d'emboîtement du connecteur mâle 10 dans le connecteur femelle 20. A l'intérieur du connecteur mâle 10, la portion rétrécie de canal 33 fait la jonction fluidique entre d'une part le tronçon 13 de canal à section également rétrécie appartenant à la première capsule 1 et d'autre part le tronçon 23 de canal à section également rétrécie appartenant à la deuxième capsule 2. Cette collerette 18 rend plus robuste le premier coude à angle droit du canal de communication inter-capsules.

Du côté de la deuxième capsule 2, le connecteur femelle 20 présente une collerette 28 située à sa base dans un plan perpendiculaire à l'axe d'emboîtement du connecteur mâle 10 dans le connecteur femelle 20. A l'intérieur du connecteur mâle 10, la portion rétrécie de canal 33 fait la jonction fluidique entre d'une part le tronçon 13 de canal à section également rétrécie appartenant à la première capsule 1 et d'autre part le tronçon 23 de canal à section également rétrécie appartenant à la deuxième capsule 2. Cette collerette 28 rend plus robuste le deuxième coude à angle droit du canal de communication inter-capsules.

La figure 8C représente un agrandissement d'une vue de coupe partielle selon l'axe BB d'un exemple de couple de capsules de la figure 8A.

Le tronçon 13 de canal à section rétrécie, la portion rétrécie de canal 33, et le tronçon 23 de canal à section rétrécie, ont tous les trois la même valeur de section rétrécie.

La largeur L4 du tronçon 13 et du tronçon 23 vaut par exemple 2.31mm. Le diamètre extérieur D1 des collerettes 18 et 28 vaut par exemple 9.30mm. Le diamètre intérieur D2 de la portion rétrécie de canal 33 vaut par exemple 1.3mm. Le diamètre extérieur D3 du connecteur femelle 20 vaut par exemple 5.30mm. La hauteur H1 du connecteur mâle 10 et du connecteur femelle 20 vaut par exemple 4.30mm. La hauteur H2 du tronçon 13 et du tronçon 23 vaut par exemple 0.75mm.

Bien entendu, la présente invention n'est pas limitée aux exemples et au mode de réalisation décrits et représentés, mais elle est susceptible de nombreuses variantes accessibles à l'homme de l'art , tant qu'elle reste dans le cadre des revendications.

## Revendications

1. Capsule déformable à usage unique comprenant :
> une poche (11) contenant une quantité prédéterminée d'une seule des deux phases dont le mélange forme un produit cosmétique prêt à l'emploi,
> un rétrécissement en forme de canal (13) débouchant d'un côté dans la poche (11, 12) et de l'autre côté à l'extérieur de la capsule (1),
> la poche (11, 12) est aplatie,
∘ l'une (17) de ses deux faces étant plane,
∘ l'autre (16) de ses deux faces étant bombée au moins en partie et souple de manière à permettre l'expulsion du contenu de la poche (11, 12) par pression mécanique sur elle,
**caractérisée en ce que**:
➢ un connecteur mâle (10) qui est en plastique moulé et qui est destiné à s'accoupler avec un connecteur femelle (20) d'une autre capsule (2),
et **en ce que** :
➢ la face (16) au moins en partie bombée de la poche (11, 12) est formée par une coque thermoformée (16) contenant ladite phase, la face plane (17) de la poche (11, 12) est formée par un film (17) recouvrant cette coque (16).

2. Capsule déformable à usage unique comprenant :
➢ une poche (21) contenant une quantité prédéterminée d'une seule des deux phases dont le mélange forme un produit cosmétique prêt à l'emploi,
➢ un rétrécissement en forme de canal (23) débouchant d'un côté dans la poche (21, 22) et de l'autre côté à l'extérieur de la capsule (2),
➢ la poche (21, 22) est aplatie,
∘ l'une (17) de ses deux faces étant plane,
∘ l'autre (16) de ses deux faces étant bombée au moins en partie et souple de manière à permettre l'expulsion du contenu de la poche (21, 22) par pression mécanique sur elle,
**caractérisée en ce que**:
➢ un connecteur femelle (20) qui est en plastique moulé et qui est destiné à s'accoupler avec un connecteur mâle (10) d'une autre capsule (1),
et **en ce que** :
➢ la face (16) au moins en partie bombée de la poche (21, 22) est formée par une coque thermoformée (16) contenant ladite phase, la face plane (17) de la poche (21, 22) est formée par un film (17) recouvrant cette coque (16).

3. Capsule déformable à usage unique selon la revendication 1 ou 2, **caractérisée en ce que** la poche (11, 12 et 21, 22) comprend deux compartiments (11, 12 et 21, 22) séparés par une paroi frangible (14, 24), ladite phase se trouvant seulement dans le compartiment (11, 21) où ne débouche pas ledit canal (13, 23), seul ce compartiment (11, 21) comprenant ladite phase étant bombé avant rupture de ladite paroi frangible (14, 24).

4. Capsule déformable à usage unique selon la revendication 1 ou 2 ou 3, **caractérisée en ce que** cette portion (10, 20) de connecteur est en polymère injecté, avantageusement en polypropylène ou en polyéthylène.

5. Capsule déformable à usage unique selon l'une quelconque des revendications précédentes, **caractérisée en ce que** le film (17) est préférentiellement soudé sur cette coque (16), avantageusement soudé par chauffage thermique.

6. Capsule déformable à usage unique selon la revendication 5, **caractérisée en ce qu'**une face (16) du canal (3, 13, 23, 33) est au moins en partie bombée, et de préférence est formée par une coque thermoformée (16), l'autre face (17) du canal (3, 13, 23, 33) est au moins en partie plane, et de préférence est formée par un film (17) recouvrant cette coque (16), le film (17) étant préférentiellement soudé sur cette coque (16), avantageusement soudé par chauffage thermique, et/ou **en ce que** la coque thermoformée (16) est multicouche et comprend avantageusement une couche interne en polymère polyoléfine, le film (17) est multicouche et comprend avantageusement une couche interne en polymère polyoléfine.

7. Capsule déformable à usage unique selon l'une quelconque des revendications précédentes, **caractérisée en ce que** la capsule (1, 2) comprend une zone tampon (12, 22) vide séparée de la poche (11, 12 et 21, 22) par une paroi frangible (14, 24), aucun constituant cosmétique ne se trouvant dans ladite zone tampon (12, 22), ledit canal (3, 13, 23, 33) débouche d'un côté au niveau de la zone tampon (12, 22) et de l'autre côté à l'extérieur de la capsule (1, 2).

8. Capsule déformable à usage unique selon l'une quelconque des revendications précédentes, **caractérisée en ce que** ledit canal (13, 23, 33) est de section constante sur la majeure partie de sa longueur, de préférence sur la totalité de sa longueur,
et de préférence **en ce que** ladite partie de canal (13, 23, 33) à section constante présente une section comprise entre 0.5 et 3 mm², de préférence entre 1 et 2 mm².

9. Capsule déformable à usage unique selon l'une quelconque des revendications précédentes, **caractérisée en ce que** ledit canal (13, 23, 33) présente une taille ou un diamètre qui vaut au plus 20% de la taille ou du diamètre de la poche (11, 12 et 21, 22), de préférence au plus 15%, encore plus de préférence au plus 12%,
et/ou **en ce que** ledit canal (13, 23, 33) présente une taille ou un diamètre qui vaut au moins 2% de la taille ou du diamètre de la poche (11, 12 et 21, 22), de préférence au moins 4%, encore plus de préférence au moins 8%.

10. Capsule déformable à usage unique selon l'une quelconque des revendications précédentes, **caractérisée en ce que** l'épaisseur de la poche (11, 12 et 21, 22) est comprise entre 5% et 30% de sa longueur, de préférence entre 10% et 20% de sa longueur,
et/ou **en ce que** l'épaisseur de la poche (11, 12 et 21, 22) est comprise entre 10% et 50% de sa largeur, de préférence entre 20% et 40% de sa largeur.

11. Couple de capsules déformables à usage unique, comprenant une capsule selon la revendication 1 ou d'une des revendications 3-10 dépendante de la revendication 1 et une capsule selon la revendication 2 ou d'une des revendications 3-10 dépendante de la revendication 2.

12. Couple de capsules déformables à usage unique comprenant :
➢ une première capsule (1) comprenant :
∘ une première poche (11, 12) contenant une quantité prédéterminée d'une seule des deux phases dont le mélange forme un produit cosmétique prêt à l'emploi,
∘ un premier rétrécissement en forme de premier canal (13) débouchant d'un côté dans la première poche (11, 12) et de l'autre côté à l'extérieur de la première capsule (1),
∘ la première poche (11, 12) étant aplatie,
▪ l'une (17) de ses deux faces étant plane,
▪ l'autre (16) de ses deux faces étant bombée au moins en partie et souple de manière à permettre l'expulsion du contenu de la première poche (11, 12) par pression mécanique sur elle,
➢ un connecteur mâle (10) qui est en plastique moulé et qui est destiné à s'accoupler avec un connecteur femelle (20) d'une autre capsule (2),
➢ la face (16) au moins en partie bombée de la poche (11, 12) est formée par une coque thermoformée (16) contenant ladite phase, la face plane (17) de la poche (11, 12) est formée par un film (17) recouvrant cette coque (16),
➢ une deuxième capsule (2) comprenant :
∘ une deuxième poche (21, 22) contenant une quantité prédéterminée de l'autre seulement des deux phases dont le mélange forme ledit produit cosmétique prêt à l'emploi,
∘ un deuxième rétrécissement en forme de deuxième canal (23) débouchant d'un côté dans la deuxième poche (21, 22) et de l'autre côté à l'extérieur de la deuxième capsule (2),
∘ la deuxième poche (21, 22) étant aplatie,
▪ l'une (17) de ses deux faces étant plane,
▪ l'autre (16) de ses deux faces étant bombée au moins en partie et souple de manière à permettre l'expulsion du contenu de la deuxième poche (2) par pression mécanique sur elle,
➢ un connecteur femelle (20) qui est en plastique moulé et qui est destiné à s'accoupler avec un connecteur mâle (10) d'une autre capsule (1),
et en ce que :
➢ la face (16) au moins en partie bombée de la poche (11, 12 et 21, 22) est formée par une coque thermoformée (16) contenant ladite phase, la face plane (17) de la poche (11, 12 et 21, 22) est formée par un film (17) recouvrant cette coque (16),
➢ la première capsule (1) et la deuxième capsule (2) étant destinées à être reliées entre elles par la mise en communication entre le premier canal (13) et le deuxième canal (23), par leur côté débouchant à l'extérieur de leur capsule (1, 2), par le connecteur mâle (10) s'emboîtant dans le connecteur femelle (20).

13. Couple de capsules déformables à usage unique selon la revendication 11 ou 12, **caractérisé en ce que** :
➢ la première capsule (1) et la deuxième capsule (2) sont reliées entre elles par la mise en communication entre le premier canal (13) et le deuxième canal (23), par leur côté débouchant à l'extérieur de leur capsule (1, 2),
➢ l'intérieur des premier (13) et deuxième canaux (23) forme un espace situé entre les première (11, 12) et deuxième (21, 22) poches et qui, étendu à l'intérieur des première (11, 12) et deuxième (21, 22) poches, est :
∘ séparé, par une première paroi frangible (14), d'un compartiment (11) de la première poche (11, 12) contenant l'une des deux dites phases,
∘ séparé, par une deuxième paroi frangible (24), d'un compartiment (21) de la deuxième poche (21, 22) contenant l'autre des deux dites phases,
∘ vide, avant la rupture des première (14) et deuxième (24) parois frangibles.

14. Couple de capsules déformables à usage unique selon l'une quelconque des revendications 11 à 13, **caractérisé en ce que** ledit rétrécissement en forme de canal (13, 23, 33) est suffisamment étroit et suffisamment long, pour qu'une pression mécanique puisse transférer le contenu d'une desdites poches (11, 12 et 21, 22) vers l'autre desdites poches (11, 12 et 21, 22), de manière à réaliser un cisaillement dudit contenu sortant dudit rétrécissement en forme du canal (13, 23, 33), ledit cisaillement homogénéisant le mélange desdites deux phases, ledit mélange homogénéisé étant un produit cosmétique directement consommable par le consommateur final.

15. Couple de capsules déformables à usage unique selon l'une quelconque des revendications 11 à 14, **caractérisé en ce que** le canal (13, 23, 33), formé par la partie à section constante du premier canal (13, 33) et la partie à section constante du deuxième canal (23, 33), présente une longueur comprise entre 0.5 et 10 mm, de préférence comprise entre 1 et 5 mm, encore plus de préférence entre 2 et 4 mm.

16. Couple de capsules déformables à usage unique selon l'une quelconque des revendications 11 à 15, **caractérisé en ce que** lesdites poches (11, 12 et 21, 22) sont chacune suffisamment volumineuse pour contenir tout ledit mélange, de préférence suffisamment volumineuse pour contenir une fois et quart tout ledit mélange, encore plus de préférence suffisamment volumineuse pour contenir une fois et demie tout ledit mélange.

17. Couple de capsules déformables à usage unique selon l'une quelconque des revendications 11 à 16, **caractérisé en ce que** ladite première poche (11, 12) à usage unique comprend une quantité prédéterminée d'une phase excipient d'un produit cosmétique, ladite deuxième poche (21, 22) à usage unique comprend une quantité prédéterminée d'une phase contenant des éléments actifs d'un produit cosmétique, ladite deuxième poche (21, 22) étant avantageusement stérilisée,
et/ou **en ce que** ladite première poche (11, 12) à usage unique comprend une quantité prédéterminée d'une phase grasse d'un produit cosmétique, ladite deuxième poche (21, 22) à usage unique comprend une quantité prédéterminée d'une phase aqueuse d'un produit cosmétique, ladite deuxième poche (21, 22) étant avantageusement stérilisée.

## Patentansprüche

1. Verformbare Einwegkapsel, umfassend:
➢ eine Tasche (11), die eine vorbestimmte Menge nur einer der beiden Phasen enthält, deren Mischung ein gebrauchsfertiges kosmetisches Produkt bildet,
➢ eine kanalförmige Verengung (13), die auf einer Seite in die Tasche (11, 12) und auf der anderen Seite außerhalb der Kapsel (1) mündet,
> wobei die Tasche (11, 12) abgeflacht ist,
∘ wobei eine (17) ihrer beiden Seiten flach ist,
∘ wobei die andere (16) ihrer beiden Seiten wenigstens teilweise gewölbt und flexibel ist, um das Herausdrücken des Inhalts der Tasche (11, 12) durch mechanischen Druck darauf zu ermöglichen,
**dadurch gekennzeichnet, dass**:
➢ ein männliches Verbindungselement (10), das aus geformtem Kunststoff besteht und das dazu bestimmt ist, mit einem weiblichen Verbindungselement (20) einer anderen Kapsel (2) zusammenzupassen,
und dadurch, dass:
➢ die wenigstens teilweise gewölbte Seite (16) der Tasche (11, 12) durch eine thermogeformte Schale (16) gebildet ist, die die Phase enthält, und die flache Seite (17) der Tasche (11, 12) durch einen Film (17) gebildet ist, der diese Schale (16) bedeckt.

2. Verformbare Einwegkapsel, umfassend:
➢ eine Tasche (21), die eine vorbestimmte Menge nur einer der beiden Phasen umfasst, deren Mischung ein gebrauchsfertiges kosmetisches Produkt bildet,
➢ eine kanalförmige Verengung (23), die auf der einen Seite in die Tasche (21, 22) und auf der anderen Seite außerhalb der Kapsel (2) mündet,
➢ wobei die Tasche (21, 22) abgeflacht ist,
∘ wobei eine (17) ihrer beiden Seiten flach ist,
∘ wobei die andere (16) ihrer beiden Seiten wenigstens teilweise gewölbt und flexibel ist, um das Herausdrücken des Inhalts der Tasche (21, 22) durch mechanischen Druck darauf zu ermöglichen,
**dadurch gekennzeichnet, dass**:
➢ ein weibliches Verbindungselement (20), das aus geformtem Kunststoff besteht und dazu bestimmt ist, mit einem männlichen Verbindungselement (10) einer anderen Kapsel (1) gekoppelt zu werden,
und dadurch, dass:
➢ die wenigstens teilweise gewölbte Seite (16) der Tasche (21, 22) durch eine thermogeformte Schale (16) gebildet ist, die die Phase enthält, und die flache Seite (17) der Tasche (21, 22) durch einen Film (17) gebildet ist, der diese Schale (16) bedeckt.

3. Verformbare Einwegkapsel nach Anspruch 1 oder 2, **dadurch gekennzeichnet, dass** die Tasche (11, 12 und 21, 22) zwei Kammern (11, 12 und 21, 22) umfasst, die durch eine brechbare Wand (14, 24) getrennt sind, wobei sich die Phase nur in der Kammer (11, 21) befindet, in die nicht der Kanal (13, 23) mündet, wobei nur diese Kammer (11, 21), die die Phase enthält, vor dem Bruch der brechbaren Wand (14, 24) gewölbt ist.

4. Verformbare Einwegkapsel nach Anspruch 1 oder 2 oder 3, **dadurch gekennzeichnet, dass** dieser Teil des Verbindungselements (10, 20) aus gespritztem Polymer, vorteilhafterweise aus Polypropylen oder Polyethylen, besteht.

5. Verformbare Einwegkapsel nach einem der vorhergehenden Ansprüche, **dadurch gekennzeichnet, dass** der Film (17) vorzugsweise auf diese Schale (16) aufgeschweißt ist, vorteilhafterweise durch thermisches Erhitzen aufgeschweißt ist.

6. Verformbare Einwegkapsel nach Anspruch 5, **dadurch gekennzeichnet, dass** eine Seite (16) des Kanals (3, 13, 23, 33) wenigstens teilweise gewölbt ist und bevorzugt durch eine thermogeformte Schale (16) gebildet ist, und die andere Seite (17) des Kanals (3, 13, 23, 33) wenigstens teilweise flach ist und bevorzugt durch einen Film (17) gebildet ist, der diese Schale (16) bedeckt, wobei der Film (17) vorzugsweise auf diese Schale (16) aufgeschweißt ist, vorteilhafterweise durch thermisches Erhitzen geschweißt, und/oder dass die thermogeformte Schale (16) mehrschichtig ist und vorteilhafterweise eine Innenschicht aus Polyolefinpolymer umfasst, wobei der Film (17) mehrschichtig ist und vorteilhafterweise eine Innenschicht aus Polyolefinpolymer umfasst.

7. Verformbare Einwegkapsel nach einem der vorhergehenden Ansprüche, **dadurch gekennzeichnet, dass** die Kapsel (1, 2) eine leere Pufferzone (12, 22) umfasst, die von der Tasche (11, 12 und 21, 22) durch eine brechbare Wand (14, 24) getrennt ist, wobei sich kein kosmetischer Bestandteil in der Pufferzone (12, 22) befindet, wobei der Kanal (3, 13, 23, 33) auf der einen Seite in der Pufferzone (12, 22) und auf der anderen Seite außerhalb der Kapsel (1, 2) mündet.

8. Verformbare Einwegkapsel nach einem der vorhergehenden Ansprüche, **dadurch gekennzeichnet, dass** der Kanal (13, 23, 33) über den größten Teil seiner Länge, bevorzugt über seine gesamte Länge, einen konstanten Querschnitt aufweist, und dass bevorzugt der Teil des Kanals (13, 23, 33) mit konstantem Querschnitt einen Querschnitt zwischen 0,5 und 3 mm², bevorzugt zwischen 1 und 2 mm², aufweist.

9. Verformbare Einwegkapsel nach einem der vorhergehenden Ansprüche, **dadurch gekennzeichnet, dass** der Kanal (13, 23, 33) eine Größe oder einen Durchmesser aufweist, der höchstens 20 % der Größe oder des Durchmessers der Tasche (11, 12 und 21, 22) beträgt, bevorzugt höchstens 15 %, noch stärker bevorzugt höchstens 12 %,
und/oder dass der Kanal (13, 23, 33) eine Größe oder einen Durchmesser aufweist, der wenigstens 2 % der Größe oder des Durchmessers der Tasche (11, 12 und 21, 22) beträgt, bevorzugt wenigstens 4 %, noch stärker bevorzugt wenigstens 8 %.

10. Verformbare Einwegkapsel nach einem der vorhergehenden Ansprüche, **dadurch gekennzeichnet, dass** die Dicke der Tasche (11, 12 und 21, 22) zwischen 5 % und 30 % ihrer Länge, bevorzugt zwischen 10 % und 20 % ihrer Länge, beträgt, und/oder dass die Dicke der Tasche (11, 12 und 21, 22) zwischen 10 % und 50 % ihrer Breite, bevorzugt zwischen 20 % und 40 % ihrer Breite, beträgt.

11. Paar verformbarer Einwegkapseln, umfassend eine Kapsel nach Anspruch 1 oder einem der Ansprüche 3 bis 10, die von Anspruch 1 abhängig sind, und eine Kapsel nach Anspruch 2 oder einem der Ansprüche 3 bis 10, die von Anspruch 2 abhängig ist.

12. Paar verformbarer Einwegkapseln, umfassend:
➢ eine erste Kapsel (1), umfassend:
∘ eine erste Tasche (11, 12), die eine vorbestimmte Menge nur einer der beiden Phasen enthält, deren Mischung ein gebrauchsfertiges kosmetisches Produkt bildet,
∘ eine erste Verengung in Form eines ersten Kanals (13), der auf einer Seite in die erste Tasche (11, 12) und auf der anderen Seite außerhalb der ersten Kapsel (1) mündet,
∘ wobei die erste Tasche (11, 12) abgeflacht ist,
▪ wobei eine (17) ihrer beiden Seiten flach ist,
▪ wobei die andere (16) der beiden Seiten wenigstens teilweise gewölbt und flexibel ist, um das Herausdrücken des Inhalts der ersten Tasche (11, 12) durch mechanischen Druck darauf zu ermöglichen,
➢ ein männliches Verbindungselement (10), das aus einem geformten Kunststoff besteht und dazu bestimmt ist, mit einem weiblichen Verbindungselement (20) einer anderen Kapsel (2) gekoppelt zu werden,
➢ wobei die wenigstens teilweise gewölbte Seite (16) der Tasche (11, 12) durch eine thermogeformte Schale (16) gebildet ist, die die Phase umfasst, und die flache Seite (17) der Tasche (11, 12) durch einen Film (17) gebildet ist, der diese Schale (16) bedeckt,
➢ eine zweite Kapsel (2), umfassend:
∘ eine zweite Tasche (21, 22), die eine vorbestimmte Menge nur der anderen der beiden Phasen enthält, deren Mischung das gebrauchsfertige kosmetische Produkt bildet,
∘ eine zweite Verengung in Form eines zweiten Kanals (23), der auf der einen Seite in die zweite Tasche (21, 22) und auf der anderen Seite außerhalb der zweiten Kapsel (2) mündet,
∘ wobei die zweite Tasche (21, 22) abgeflacht ist,
▪ wobei eine (17) ihrer beiden Seiten flach ist,
▪ wobei die andere (16) ihrer beiden Seiten wenigstens teilweise gewölbt und flexibel ist, um das Herausdrücken des Inhalts der zweiten Tasche (2) durch mechanischen Druck darauf zu ermöglichen,
➢ ein weibliches Verbindungselement (20), das aus geformtem Kunststoff besteht und das dazu bestimmt ist, mit einem männlichen Verbindungselement (10) einer anderen Kapsel (1) gekoppelt zu werden,
und dadurch, dass:
➢ die wenigstens teilweise gewölbte Seite (16) der Tasche (11, 12 und 21, 22) durch eine thermogeformte Schale (16) gebildet ist, die die Phase umfasst, wobei die flache Seite (17) der Tasche (11, 12 und 21, 22) durch einen Film (17) gebildet ist, der diese Schale (16) bedeckt,
➢ die erste Kapsel (1) und die zweite Kapsel (2) dazu bestimmt sind, miteinander verbunden zu werden, indem der erste Kanal (13) und der zweite Kanal (23) auf ihrer Seite, die außerhalb der Kapsel (1, 2) mündet, durch das männliche Verbindungselement (10), das in das weibliche Verbindungselement (20) eingesteckt ist, miteinander in Verbindung gebracht werden.

13. Paar verformbarer Einwegkapseln nach Anspruch 11 oder 12, **dadurch gekennzeichnet, dass**:
➢ die erste Kapsel (1) und die zweite Kapsel (2) durch die Verbindung zwischen dem ersten Kanal (13) und dem zweiten Kanal (23) auf ihrer Seite, die außerhalb ihrer Kapsel (1, 2) mündet, miteinander verbunden sind,
➢ das Innere des ersten (13) und des zweiten (23) Kanals einen zwischen der ersten (11, 12) und der zweiten (21, 22) Tasche gelegenen Raum bildet, der, wenn er sich innerhalb der ersten (11, 12) und zweiten (21, 22) Tasche erstreckt:
o durch eine erste brechbare Wand (14) von einer Kammer (11) der ersten Tasche (11, 12), die eine der beiden Phasen umfasst, getrennt ist,
∘ durch eine zweite brechbare Wand (24) von einer Kammer (21) der zweiten Tasche (21, 22), die die andere der beiden Phasen enthält, getrennt ist,
∘ leer ist, bevor die erste (14) und die zweite (24) brechbare Wand brechen.

14. Paar verformbarer Einwegkapseln nach einem der Ansprüche 11 bis 13, **dadurch gekennzeichnet, dass** die kanalförmige Verengung (13, 23, 33) ausreichend schmal und lang ist, so dass ein mechanischer Druck den Inhalt einer der Taschen (11, 12 und 21, 22) in die andere der Taschen (11, 12 und 21, 22) überführen kann, um eine Scherung des Inhalts zu bewirken, der aus der kanalförmigen Verengung (13, 23, 33) austritt, wobei die Scherung die Mischung der beiden Phasen homogenisiert, wobei die homogenisierte Mischung ein kosmetisches Produkt ist, das vom Endverbraucher direkt verbraucht werden kann.

15. Paar verformbare Einwegkapseln nach einem der Ansprüche 11 bis 14, **dadurch gekennzeichnet, dass** der Kanal (13, 23, 33), der durch den Teil mit konstantem Querschnitt des ersten Kanals (13, 33) und den Teil mit konstantem Querschnitt des zweiten Kanals (23, 33) gebildet ist, eine Länge zwischen 0,5 und 10 mm, bevorzugt zwischen 1 und 5 mm, stärker bevorzugt zwischen 2 und 4 mm, aufweist.

16. Paar verformbarer Einwegkapseln nach einem der Ansprüche 11 bis 15, **dadurch gekennzeichnet, dass** die Taschen (11, 12 und 21, 22) jeweils ausreichend groß sind, um die gesamte Mischung zu umfassen, bevorzugt ausreichend groß, um eineinviertel Mal die gesamte Mischung zu umfassen, stärker bevorzugt ausreichend groß, um eineinhalb Mal die gesamte Mischung zu enthalten.

17. Paar verformbarer Einwegkapseln nach einem der Ansprüche 11 bis 16, **dadurch gekennzeichnet, dass** die erste Einwegtasche (11, 12) eine vorbestimmte Menge einer Trägerphase eines kosmetischen Produkts umfasst, die zweite Einwegtasche (21, 22) eine vorbestimmte Menge einer Phase umfasst, die aktive Elemente eines kosmetischen Produkts enthält, wobei die zweite Tasche (21, 22) vorteilhafterweise sterilisiert ist,
und/oder dass die erste Einwegtasche (11, 12) eine vorbestimmte Menge einer Fettphase eines kosmetischen Produkts umfasst, die zweite Einwegtasche (21, 22) eine vorbestimmte Menge einer wässrigen Phase eines kosmetischen Produkts umfasst, wobei die zweite Tasche (21, 22) vorteilhafterweise sterilisiert ist.

## Claims

1. Disposable deformable capsule comprising:
➢ a pouch (11) containing a predetermined amount of only one of two phases which when mixed form a ready-to-use cosmetic product,
➢ a narrowing which forms a channel (13) leading at one side to the pouch (11, 12) and at the other side to outside the capsule (1),
➢ the pouch (11, 12) is flattened,
∘ one (17) of its two faces being flat,
∘ the other (16) of its two faces being at least partly convex and flexible to allow expelling the contents of the pouch (11, 12) by mechanical pressure on said pouch,
**characterized in that**:
➢ a male connector (10) which is made of molded plastic and which is intended to mate with a female connector (20) of another capsule (2),
and **in that**:
➢ the at least partly convex face (16) of the pouch (11, 12) is formed by a thermoformed shell (16) containing said phase, the flat face (17) of the pouch (11, 12) is formed by a film (17) covering this shell (16).

2. Disposable deformable capsule comprising:
➢ a pouch (21) containing a predetermined amount of only one of two phases which when mixed form a ready-to-use cosmetic product,
➢ a narrowing which forms a channel (23) leading at one side to the pouch (21, 22) and at the other side to outside the capsule (2),
➢ the pouch (21, 22) is flattened,
∘ one (17) of its two faces being flat,
∘ the other (16) of its two faces being at least partly convex and flexible to allow expelling the contents of the pouch (21, 22) by mechanical pressure on said pouch,
**characterized in that**:
➢ a female connector (20) which is made of molded plastic and which is intended to mate with a male connector (10) of another capsule (1),
and **in that**:
➢ the at least partly convex face (16) of the pouch (21, 22) is formed by a thermoformed shell (16) containing said phase, the flat face (17) of the pouch (21, 22) is formed by a film (17) covering this shell (16).

3. Disposable deformable capsule according to claim 1 or 2, **characterized in that** the pouch (11, 12 and 21, 22) comprises two compartments (11, 12 and 21, 22) separated by a frangible wall (14, 24), said phase being located only in the compartment (11, 21) not opening into said channel (13, 23), only this compartment (11, 21) comprising said phase being convex prior to rupture of said frangible wall (14, 24).

4. Disposable deformable capsule according to claim 1 or 2 or 3, **characterized in that** this connector part (10, 20) is made of injected polymer, advantageously polypropylene or polyethylene.

5. Disposable deformable capsule according to any of the preceding claims, **characterized in that** the film (17) is preferably welded onto this shell (16), advantageously welded by thermal heating.

6. Disposable deformable capsule according to claim 5, **characterized in that** one face (16) of the channel (3, 13, 23, 33) is at least partly convex, and preferably is formed by a thermoformed shell (16), the other face (17) of the channel (3, 13, 23, 33) is at least partly flat, and preferably is formed by a film (17) covering this shell (16), the film (17) preferably being welded to this shell (16), advantageously welded by thermal heating,
and/or **in that** the thermoformed shell (16) is multilayer and advantageously comprises an inner layer of polyolefin polymer, and the film (17) is multilayer and advantageously comprises an inner layer of polyolefin polymer.

7. Disposable deformable capsule according to any one of the preceding claims, **characterized in that** the capsule (1, 2) comprises an empty buffer zone (12, 22) separated from the pouch (11, 12 and 21, 22) by a frangible wall (14, 24), no cosmetic component being in said buffer zone (12, 22), and said channel (3, 13, 23, 33) leads at one side to the buffer zone (12, 22) and at the other side to outside the capsule (1, 2).

8. Disposable deformable capsule according to any one of the preceding claims, **characterized in that** said channel (13, 23, 33) is of constant cross-section along most of its length, preferably its entire length,
and preferably **in that** said channel portion (13, 23, 33) of constant cross-section has a cross-section of between 0.5 and 3 mm², preferably between 1 and 2 mm².

9. Disposable deformable capsule according to any one of the preceding claims, **characterized in that** said channel (13, 23, 33) has a size or diameter which is at most 20% of the size or diameter of the pouch (11, 12 and 21, 22), preferably at most 15%, more preferably at most 12%, and/or **in that** said channel (13, 23, 33) has a size or diameter which is at least 2% of the size or diameter of the pouch (11, 12 and 21, 22), preferably at least 4%, more preferably at least 8%.

10. Disposable deformable capsule according to any one of the preceding claims, **characterized in that** the thickness of the pouch (11, 12 and 21, 22) is between 5% and 30% of its length, preferably between 10% and 20% of its length,
and/or the thickness of the pouch (11, 12 and 21, 22) is between 10% and 50% of its width, preferably between 20% and 40% of its width.

11. Pair of disposable deformable capsules, comprising a capsule according to claim 1 or any one of claims 3-10 dependent on claim 1 and a capsule according to claim 2 or any one of claims 3-10 dependent on claim 2.

12. Pair of disposable deformable capsules comprising:
➢ a first capsule (1) comprising:
∘ a first pouch (11, 12) containing a predetermined amount of only one of two phases which when mixed form a ready-to-use cosmetic product,
∘ a first narrowing which forms a channel (13) leading at one side to the first pouch (11, 12) and at the other side to outside the first capsule (1),
∘ the first pouch (11, 12) being flattened,
▪ one (17) of its two faces being flat,
▪ the other (16) of its two faces being at least partly convex and flexible to allow expelling the contents of the first pouch (11, 12) by mechanical pressure on said pouch,
➢ a male connector (10) which is made of molded plastic and which is intended to mate with a female connector (20) of another capsule (2),
➢ the at least partly convex face (16) of the pouch (11, 12) is formed by a thermoformed shell (16) containing said phase, the flat face (17) of the pouch (11, 12) is formed by a film (17) covering this shell (16),
➢ a second capsule (2) comprising:
∘ a second pouch (21, 22) containing a predetermined amount of only the other of the two phases which when mixed form said ready-to-use cosmetic product,
∘ a second narrowing which forms a second channel (23) leading at one side to the second pouch (21, 22) and at the other side to outside the second capsule (2),
∘ the second pouch (21, 22) being flattened,
▪ one (17) of its two faces being flat,
▪ the other (16) of its two faces being at least partly convex and flexible to allow expelling the contents of the second pouch (2) by mechanical pressure on said pouch,
➢ a female connector (20) which is made of molded plastic and which is intended to mate with a male connector (10) of another capsule (1),
and in that:
➢ the at least partly convex face (16) of the pouch (11, 12 and 21, 22) is formed by a thermoformed shell (16) containing said phase, the flat face (17) of the pouch (11, 12 and 21, 22) is formed by a film (17) covering this shell (16),
➢ the first capsule (1) and the second capsule (2) are intended to be interconnected by the communication established between the first channel (13) and the second channel (23), by their side which leads to outside their capsule (1, 2), by the male connector (10) fitting into the female connector (20).

13. Pair of disposable deformable capsules according to claim 11 or 12, **characterized in that**:
➢ the first capsule (1) and the second capsule (2) are interconnected by the communication established between the first channel (13) and the second channel (23), by their side which leads to outside their capsule (1, 2),
➢ the interior of the first (13) and second channels (23) forms a space located between the first (11, 12) and second (21, 22) pouches and which, extending to inside the first (11, 12) and second (21, 22) pouches, is:
∘ separated, by a first frangible wall (14), from a compartment (11) of the first pouch (11, 12) containing one of said two phases,
∘ separated, by a second frangible wall (24), from a compartment (21) of the second pouch (21, 22) containing the other of said two phases,
∘ empty before the first (14) and second (24) frangible walls are ruptured.

14. Pair of disposable deformable capsules according to any one of claims 11 to 13, **characterized in that** said narrowing which forms a channel (13, 23, 33) is sufficiently narrow and sufficiently long for mechanical pressure to be able to transfer the contents of one of said pouches (11, 12 and 21, 22) to the other of said pouches (11, 12 and 21, 22), creating shear in said contents exiting said narrowing which forms a channel (13, 23, 33), said shear homogenizing the mixture of said two phases, said homogenized mixture being a cosmetic product directly consumable by the end consumer.

15. Pair of disposable deformable capsules according to any one of claims 11 to 14, **characterized in that** the channel (13, 23, 33) formed by the portion of constant cross-section of the first channel (13, 33) and the portion of constant cross-section of the second channel (23, 33) has a length of between 0.5 and 10 mm, preferably between 1 and 5 mm, more preferably between 2 and 4 mm.

16. Pair of disposable deformable capsules according to any one of claims 11 to 15, **characterized in that** said pouches (11, 12 and 21, 22) are each sufficiently large to contain all of said mixture, preferably sufficiently large to contain one and a fourth of all of said mixture, even more preferably sufficiently large to contain one and a half of all of said mixture.

17. Pair of disposable deformable capsules according to any one of claims 11 to 16, **characterized in that** said first disposable pouch (11, 12) comprises a predetermined amount of an excipient phase of a cosmetic product, said second disposable pouch (21, 22) comprises a predetermined amount of a phase containing active ingredients of a cosmetic product, said second pouch (21, 22) advantageously being sterilized,
and/or **in that** said first disposable pouch (11, 12) comprises a predetermined amount of a fatty phase of a cosmetic product, said second disposable pouch (21, 22) comprises a predetermined amount of an aqueous phase of a cosmetic product, said second pouch (21, 22) advantageously being sterilized.
